# EUROPEAN PATENT APPLICATION

(11) **EP 0 778 267 A1**
(43) Date of publication of application: **11.06.1997**
(21) Application number: 96916333.6
(22) Date of filing: 06.06.1996
(51) Int. Cl.: C07C 311/21, C07C 311/29, C07D 213/42, A01N 41/06, A01N 43/40

(54) **SULFONAMIDE DERIVATIVES AND INSECTICIDE, MITICIDE AND NEMATICIDE CONTAINING THE SAME**

(30) Priority: 21.06.1995 JP 154484/95; 19.12.1995 JP 330076/95
(71) Applicant: OTSUKA KAGAKU KABUSHIKI KAISHA, Osaka-shi, Osaka 540 (JP)
(72) Inventor: NAKAGAWA, Hirohumi 81, Azashimokirai Shimobun, Tokushima 771-34 (JP); GOTODA, Satoshi 724, Oazakawashima, Tokushima 779-33 (JP); MURAI, Keizaburo 1-49, Azahirotsuka, Tokushima 779-02 (JP); WAKISAKA, Shigekazu, Tokushima 772 (JP); TAKAO, Hilsashi 135-11, Azahara, Tokushima 771-02 (JP)
(74) Representative: Barz, Peter, Dr.
(86) International application number: JP9601545
(87) International publication number: WO9700857

(57) **Abstract**

An object of the present invention is to provide a sulfonamide derivative which is useful as insecticidal, miticidal and nematocidal agents. The sulfonamide derivative of the invention is represented by the formula 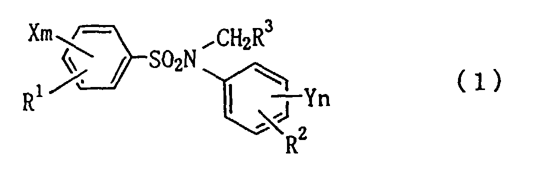 wherein R¹ and R² are the same or different and each represents a hydrogen atom, a lower haloalkyl group or a halogen atom, R³ is an ethynyl group, a cyano group, a lower alkoxy group, or a lower alkoxycarbonyl group, X and Y are the same or different and each represents a hydrogen atom, a halogen atom, a lower alkyl group, a lower alkoxy group, a lower haloalkyl group, a lower haloalkoxy group, a phenoxy group optionally having a substituent(s), a phenyl group optionally having a substituent(s), a pyridyloxy group optionally having a substituent(s), a cyano group, a nitro group, a lower alkylthio group or a lower alkyl sulfonyl group, and m and n are each an integer of 1 to 3.

## Description

### Field of the Invention

The present invention relates to sulfonamide derivatives and insecticidal, miticidal and nematocidal compositions containing the derivatives.

### Background Art

Of sulfonamide derivatives, compounds having a bioactivity are known. For example, Japanese Unexamined Patent Publication No. Hei 3-258,771 discloses that a sulfonamide derivative having a 1,2,4-oxadiazole group shows a herbicidal activity. However, said publication neither describes nor suggests that the compound disclosed therein exhibits an insecticidal activity, miticidal activity and nematocidal activity. The specification of French Patent No.957,493 discloses that a sulfonamide derivative represented by the formula 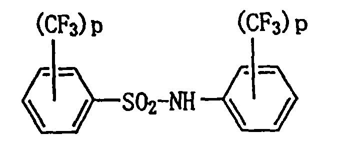 wherein p is an integer of 1 to 3 has a miticidal activity. However, no teaching is found in the specification of the French patent as to whether the sulfonamide derivative of said formula shows other bioactivity than miticidal activity, such as insecticidal activity and nematocidal activity.

### Disclosure of the Invention

An object of the present invention is to provide a composition having a high bioactivity for agricultural use.

Another object of the invention is to provide a sulfonamide derivative having a high insecticidal activity against noxious insects which pose problems from an agricultural viewpoint.

A further object of the invention is to provide a sulfonamide derivative having a potent miticidal activity and nematocidal activity.

Other features of the invention will become apparent from the following description.

The present inventors conducted various researches on sulfonamide derivatives to develop a composition having a high bioactivity for agricultural use, and found that a compound represented by the formula (1) shown below exhibits a high insecticidal activity against noxious insects which are problematic from an agricultural viewpoint, and that the compound also has high miticidal and nematocidal activities. The present invention was completed based on these novel findings.

The sulfonamide derivative of the invention is a novel compound undisclosed in literature and is represented by the formula 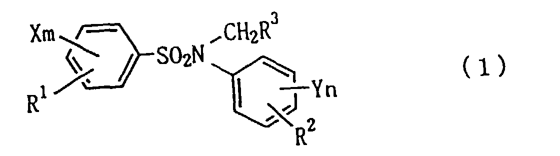 wherein R¹ and R² are the same or different and each represents a hydrogen atom, a lower haloalkyl group or a halogen atom, R³ is an ethynyl group, a cyano group, a lower alkoxy group, or a lower alkoxycarbonyl group, X and Y are the same or different and each represents a hydrogen atom, a halogen atom, a lower alkyl group, a lower alkoxy group, a lower haloalkyl group, a lower haloalkoxy group, a phenoxy group optionally having a substituent(s), a phenyl group optionally having a substituent(s), a pyridyloxy group optionally having a substituent(s), a cyano group, a nitro group, a lower alkylthio group or a lower alkyl sulfonyl group, and m and n are each an integer of 1 to 3.

The sulfonamide derivative of the invention has a high insecticidal activity and also high miticidal and nematocidal activities and is useful as insecticidal, miticidal or nematocidal agent.

Specific examples of groups described in the specification are as follows.

Examples of the lower haloalkyl group are straight-chain or branched-chain alkyl groups of 1 to 6 carbon atoms which have 1 to 3 halogen atoms as a substituent(s), such as trifluoromethyl, trichloromethyl, chloromethyl, bromomethyl, fluoromethyl, iodomethyl, difluoromethyl, dibromomethyl, 2-chloroethyl, 2,2,2-trifluoroethyl, 2,2,2-trichloroethyl, 3-bromopropyl, 3-chloropropyl, 2,3-dichloropropyl, 4,4,4-trichlorobutyl, 4-fluorobutyl, 5-chloropentyl, 3-chloro-2-methylpropyl, 5-bromohexyl, 5,6-dichlorohexyl, etc.

Examples of the halogen atom are a fluorine atom, chlorine atom, bromine atom and iodine atom.

Examples of the lower alkoxy group are straight-chain or branched-chain alkoxy groups of 1 to 6 carbon atoms, such as methoxy, ethoxy, propoxy, isopropoxy, butoxy, tert-butoxy, pentyloxy, hexyloxy, etc.

Examples of the lower alkoxycarbonyl group are straight-chain or branched-chain alkoxycarbonyl groups of 1 to 6 carbon atoms, such as methoxycarbonyl, ethoxycarbonyl, propoxycarbonyl, isopropoxycarbonyl, butoxycarbonyl, isobutoxycarbonyl, tert-butoxycarbonyl, etc.

Examples of the lower alkyl group are straight-chain or branched-chain alkyl groups of 1 to 6 carbon atoms, such as methyl, ethyl, propyl, isopropyl, butyl, isobutyl, tert-butyl, pentyl, hexyl, etc.

Examples of the lower haloalkoxy group are straight-chain or branched-chain alkoxy groups of 1 to 6 carbon atoms which have 1 to 3 halogen atoms as a substituent(s), such as trifluoromethoxy, trichloromethoxy, chloromethoxy, bromomethoxy, fluoromethoxy, iodomethoxy, difluoromethoxy, dibromomethoxy, 2-chloroethoxy, 2,2,2-trifluoroethoxy, 2,2,2-trichloroethoxy, 3-bromopropoxy, 3-chloropropoxy, 2,3-dichloropropoxy, 4,4,4-trichlorobutoxy, 4-fluorobutoxy, 5-chloropentyloxy, 3-chloro-2-methylpropoxy, 5-bromohexyloxy, 5,6-dichlorohexyloxy, etc.

Examples of the phenoxy group optionally having a substituent(s) are phenoxy groups optionally having 1 to 3 substituents on the phenyl ring, the substituent being selected from the group consisting of a straight-chain or branched-chain alkyl group of 1 to 6 carbon atoms, straight-chain or branched-chain alkoxy group of 1 to 6 carbon atoms, hydroxyl group, straight-chain or branched-chain alkanoyloxy group of 1 to 6 carbon atoms, straight-chain or branched-chain alkoxy group of 1 to 6 carbon atoms having 1 to 3 halogen atoms as a substituent, nitro group, amino group optionally having as a substituent a straight-chain or branched-chain alkanoyl group of 1 to 6 carbon atoms, phenyl group, and straight-chain or branched-chain alkoxy group of 1 to 6 carbon atoms having an amino group which may have as a substituent(s) 1 or 2 straight-chain or branched-chain alkyl groups of 1 to 6 carbon atoms, such as phenoxy, 2-methylphenoxy, 3-methylphenoxy, 4-methylphenoxy, 2-ethylphenoxy, 3-propylphenoxy, 4-butylphenoxy, 2-pentylphenoxy, 3-hexylphenoxy, 3,4-dimethylphenoxy, 3,4,5-trimethylphenoxy, 2-methoxyphenoxy, 3-methoxyphenoxy, 4-methoxyphenoxy, 2-ethoxyphenoxy, 4-ethoxyphenoxy, 3-propoxyphenoxy, 4-propoxyphenoxy, 4-butoxyphenoxy, 2-pentyloxyphenoxy, 3-hexyloxyphenoxy, 2,4-dimethoxyphenoxy, 3,4-diethoxyphenoxy, 3,4,5-trimethoxyphenoxy, 2-hydroxyphenoxy, 3-hydroxyphenoxy, 4-hydroxyphenoxy, 2,4-dihydroxyphenoxy, 3,4-dihydroxyphenoxy, 2,4,6-trihydroxyphenoxy, 2-acetyloxyphenoxy, 3-propionyloxyphenoxy, 2-benzyloxyphenoxy, 3-benzyloxyphenoxy, 4-benzyloxyphenoxy, 2-(2-phenylethoxy)phenoxy, 3-(3-phenylpropoxy)phenoxy, 4-(4-phenylbutoxy)phenoxy, 3-(1-phenylethoxy)phenoxy, 2-(5-phenylpentyloxy)phenoxy, 3-(6-phenylhexyloxy)phenoxy, 2,4-dibenzyloxyphenoxy, 3,4-dibenzyloxyphenoxy, 3,4,5-tribenzyloxyphenoxy, 4-butyryloxyphenoxy, 2-pentanoyloxyphenoxy, 4-hexanoyloxyphenoxy, 2,4-diacetyloxyphenoxy, 2,6-diacetyloxyphenoxy, 3,4,5-triacetyloxyphenoxy, 2-trifluoromethoxyphenoxy, 3-(2-chloroethoxy)phenoxy, 2-(3-bromopropoxy)phenoxy, 4-iodomethoxyphenoxy, 2-(2,3-dichloropropoxy)phenoxy, 3-(4-fluorobutoxy)phenoxy, 4-(3-chloro-2-methylpropoxy)phenoxy, 2-(5-bromohexyloxy)phenoxy, 3-(5,6-dichlorohexyloxy)phenoxy, 4-(2,2,2-trichloroethoxy)phenoxy, 2,4-bis(trifluoromethoxy)phenoxy, 2,4,6-tris(trifluoromethoxy)phenoxy, 2-aminomethoxyphenoxy, 3-(1-aminoethoxy)phenoxy, 4-(3-aminopropoxy)phenoxy, 2-(4-aminobutoxy)phenoxy, 3-(5-aminopentyloxy)phenoxy, 4-(6-aminohexyloxy)phenoxy, 2-methylaminomethoxyphenoxy, 3-(2-propylaminoethoxy)phenoxy, 2-(3-isopropylaminopropoxy)phenoxy, 4-(4-butylaminobutoxy)phenoxy, 2-(5-pentylaminopentyloxy)phenoxy, 3-(6-hexylaminohexyloxy)phenoxy, 3-(6-hexylaminohexyloxy)phenoxy, 4-dimethylaminomethoxyphenoxy, 2-(N-ethyl-N-propoylaminomethoxy)phenoxy, 2-methyl-4-methoxyphenoxy, 2-methyl-6-hydroxyphenoxy, 4-methyl-2-(3-bromopropoxy)phenoxy, 4-methoxy-2-(3-isopropylamino-propoxy)phenoxy, 2-phenylphenoxy, 3-phenylphenoxy, 4-phenylphenoxy, 2-nitrophenoxy, 3-nitrophenoxy, 4-nitrophenoxy, 2,3-dinitrophenoxy, 2,4,6-trinitrophenoxy, 2-aminophenoxy, 3-aminophenoxy, 4-aminophenoxy, 2,4-diaminophenoxy, 3,4,5-triaminophenoxy, 4-acetylaminophenoxy, 2-propionylaminophenoxy, 3-butyrylaminophenoxy, 4-pentanoylaminophenoxy, 4-hexanoylaminophenoxy, 2,3-diacetylaminophenoxy, 2,4,6-triacetylaminophenoxy, etc.

Examples of the phenyl group optionally having a substituent(s) are phenyl groups optionally having 1 to 3 substituents on the phenyl ring, the substituent being selected from the group consisting of a straight-chain or branched-chain alkyl group of 1 to 6 carbon atoms, straight-chain or branched-chain alkoxy group of 1 to 6 carbon atoms, hydroxyl group, straight-chain or branched-chain alkanoyloxy group of 1 to 6 carbon atoms, straight-chain or branched-chain alkoxy group of 1 to 6 carbon atoms having 1 to 3 halogen atoms as a substituent(s), nitro group, amino group optionally having as a substituent(s) a straight-chain or branched-chain alkanoyl group of 1 to 6 carbon atoms, a phenyl group, and a straight-chain or branched-chain alkoxy group of 1 to 6 carbon atoms having an amino group which may have as a substituent(s) 1 or 2 straight-chain or branched-chain alkyl groups of 1 to 6 carbon atoms, such as phenyl, 2-methylphenyl, 3-methylphenyl, 4-methylphenyl, 2-ethylphenyl, 3-propylphenyl, 4-butylphenyl, 2-pentylphenyl, 3-hexylphenyl, 3,4-dimethylphenyl, 3,4,5-trimethylphenyl, 2-methoxyphenyl, 3-methoxyphenyl, 4-methoxyphenyl, 2-ethoxyphenyl, 4-ethoxyphenyl, 3-propoxyphenyl, 4-propoxyphenyl, 4-butoxyphenyl, 2-pentyloxyphenyl, 3-hexyloxyphenyl, 2,4-dimethoxyphenyl, 3,4-diethoxyphenyl, 3,4,5-trimethoxyphenyl, 2-hydroxyphenyl, 3-hydroxyphenyl, 4-hydroxyphenyl, 2,4-dihydroxyphenyl, 3,4-dihydroxyphenyl, 2,4,6-trihydroxyphenyl, 2-acetyloxyphenyl, 3-propionyloxyphenyl, 2-benzyloxyphenyl, 3-benzyloxyphenyl, 4-benzyloxyphenyl, 2-(2-phenylethoxy)phenyl, 3-(3-phenylpropoxy)phenyl, 4-(4-phenylbutoxy)phenyl, 3-(1-phenylethoxy)phenyl, 2-(5-phenylpentyloxy)phenyl, 3-(6-phenylhexyloxy)phenyl, 2,4-dibenzyloxyphenyl, 3,4-dibenyloxyphenyl, 3,4,5-tribenzyloxyphenyl, 4-butyryloxyphenyl, 2-pentanoyloxyphenyl, 4-hexanoyloxyphenyl, 2,4-diacetyloxyphenyl, 2,6-diacetyloxyphenyl, 3,4,5-triacetyloxyphenyl, 2-trifluoromethoxyphenyl, 3-(2-chloroethoxy)phenyl, 2-(3-bromoproxy)phenyl, 4-iodomethoxyphenyl, 2-(2,3-dichloropropoxy)phenyl, 3-(4-fluorobutoxy)phenyl, 4-(3-chloro-2-methylpropoxy)phenyl, 2-(5-bromohexyloxy)phenyl, 3-(5,6-dichlorohexyloxy)phenyl, 4-(2,2,2-trichloroethoxy)phenyl, 2,4-bis(trifluoromethoxy)phenyl, 2,4,6-tris(trifluoromethoxy)phenyl, 2-aminomethoxyphenyl, 3-(1-aminoethoxy)phenyl, 4-(3-aminopropoxy)phenyl, 2-(4-aminobutoxy)phenyl, 3-(5-aminopentyloxy)phenyl, 4-(6-aminohexyloxy)phenyl, 2-methylaminomethoxyphenyl, 3-(2-propylaminoethoxy)phenyl, 2-(3-isopropylamino-propoxy)phenyl, 4-(4-butylamino-butoxy)phenyl, 2-(5-pentylaminopentyloxy)phenyl, 3-(6-hexylaminohexyloxy)phenyl, 4-dimethylaminomethoxyphenyl, 2-(N-ethyl-N-propylaminomethoxy)phenyl, 2-methyl-4-methoxyphenyl, 2-methyl-6-hydroxyphenyl, 4-methyl-2-(3-bromopropoxy)phenyl, 4-methoxy-2-(3-isopropylamino-propoxy)phenyl, 2-phenylphenyl, 3-phenylphenyl, 4-phenylphenyl, 2-nitrophenyl, 3-nitrophenyl, 4-nitrophenyl, 2,3-dinitrophenyl, 2,4,6-trinitrophenyl, 2-aminoethyl, 3-aminophenyl, 4-aminophenyl, 2,4-diaminophenyl, 3,4,5-triaminophenyl, 4-acetylaminophenyl, 2-propionylaminophenyl, 3-butyrylaminophenyl, 4-pentanoylaminophenyl, 4-hexanoylaminophenyl, 2,3-diacetylaminophenyl, 2,4,6-triacetylaminophenyl, etc.

Examples of the pyridyloxy group optionally having a substituent(s) are pyridyloxy groups optionally having 1 to 3 substituents on the pyridine ring, the substituent being selected from the group consisting of a straight-chain or branched-chain alkyl group of 1 to 6 carbon atoms and halogen atom, such as 2-pyridyloxy, 3-pyridyloxy, 4-pyridyloxy, 3-methyl-2-pyridyloxy, 4-methyl-2-pyridyloxy, 5-methyl-2-pyridyloxy, 2-methyl-3-pyridyloxy, 4-methyl-3-pyridyloxy, 5-methyl-3-pyridyloxy, 2-methyl-4-pyridyloxy, 3-methyl-4-pyridyloxy, 3-butyl-2-pyridyloxy, 4-butyl-2-pyridyloxy, 5-butyl-2-pyridyloxy, 2-butyl-3-pyridyloxy, 4-butyl-3-pyridyloxy, 5-butyl-3-pyridyloxy, 2-butyl-4-pyridyloxy, 3-butyl-4-pyridyloxy, 3-trifluoromethyl-2-pyridyloxy, 4-trifluoromethyl-2-pyridyloxy, 5-trifluoromethyl-2-pyridyloxy, 2-trifluoromethyl-3-pyridyloxy, 4-trifluoromethyl-3-pyridyloxy, 5-trifluoromethyl-3-pyridyloxy, 2-trifluoromethyl-4-pyridyloxy, 3-trifluoromethyl-4-pyridyloxy, 3-trichloromethyl-2-pyridyloxy, 4-trichloromethyl-2-pyridyloxy, 5-trichloromethyl-2-pyridyloxy, 2-trichloromethyl-3-pyridyloxy, 4-trichloromethyl-3-pyridyloxy, 5-trichloromethyl-3-pyridyloxy, 2-trichloromethyl-4-pyridyloxy, 3-trichloromethyl-4-pyridyloxy, etc.

Examples of the lower alkylthio group are straight-chain or branched-chain alkylthio groups of 1 to 6 carbon atoms, such as methylthio, ethylthio, propylthio, isopropylthio, butylthio, tert-butylthio, pentylthio, hexylthio, etc.

Examples of the lower alkylsulfonyl group are straight-chain or branched-chain alkylsulfonyl groups of 1 to 6 carbon atoms, such as methylsulfonyl, ethylsulfonyl, propylsulfonyl, isopropylsulfonyl, butylsulfonyl, tert-butylsulfonyl, pentylsulfonyl, hexylsulfonyl, etc.

The foregoing sulfonamide derivatives of the formula (1) include the sulfonamide derivatives represented by the formula (1a) shown below and the sulfonamide derivatives represented by the formula (1b) shown below: 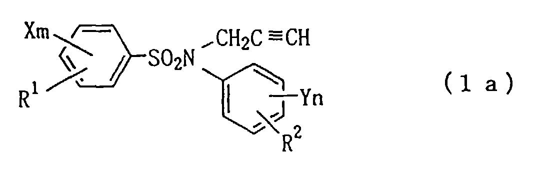 wherein R¹, R², X, Y, m and n are as defined above, 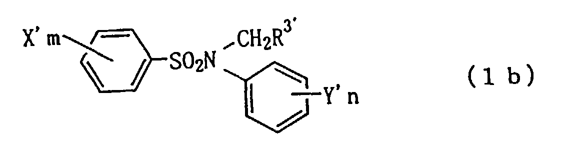 wherein R^{3'} is a cyano group, a lower alkoxy group or a lower alkoxycarbonyl group, X' is a hydrogen atom, a halogen atom, a lower haloalkyl group or a cyano group, Y' is a hydrogen atom, a halogen atom, a lower haloalkyl group, a cyano group or a nitro group, and m and n are as defined above.

Typical examples of the sulfonamide derivatives of the formula (1a) are as follows:
compounds of the formula (1a) wherein X is a halogen atom, Y is a lower haloalkyl group, R¹ and R² are hydrogen atoms, m is an integer of 1 to 3 and n is 1 or 2;
compounds of the formula (1a) wherein X is a halogen atom, Y is a lower haloalkyl group, R¹ is a hydrogen atom, R² is a halogen atom, m is an integer of 1 to 3 and n is 1 or 2;
compounds of the formula (1a) wherein X is a cyano group, Y is a lower haloalkyl group, R¹ and R² are halogen atoms, m is 1 and n is 1 or 2;
compounds of the formula (1a) wherein X is a cyano group, Y is a lower haloalkyl group, R¹ is a hydrogen atom, R² is a halogen atom, m is 1 and n is 1 or 2; and
compounds of the formula (1a) wherein X is a lower haloalkyl group, Y is a lower haloalkyl group, R¹ and R² are hydrogen atoms, and m and n are 1 or 2.

Typical examples of the sulfonamide derivatives of the formula (1b) are as follows:
compounds of the formula (1b) wherein X' is a halogen atom, Y is a lower haloalkyl group, m is 1 to 3 and n is 1 or 2;
compounds of the formula (1b) wherein X' is a lower haloalkyl group, Y' is a lower haloalkyl group, and m and n are 1 or 2;
compounds of the formula (1b) wherein R^{3'} is a cyano group; and
compounds of the formula (1b) wherein R^{3'} is a lower alkoxy group.

The sulfonamide derivatives of the present invention can be prepared, for example, by the process illustrated below in Reaction Scheme-1: 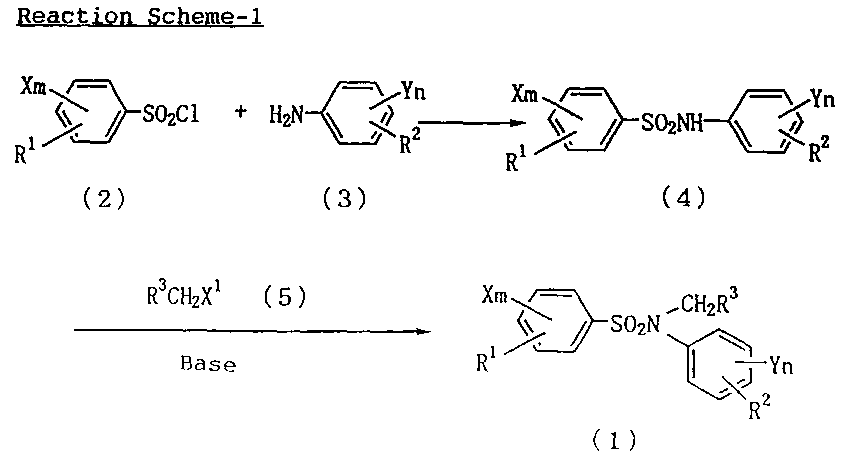 wherein R¹, R², R³, X, Y, m and n are as defined above, and X¹ is a halogen atom.

According to Reaction Scheme-1, the compound of the present invention represented by the formula (1) can be prepared by reacting a sulfonyl chloride of the formula (2) with an aniline of the formula (3) and reacting the obtained sulfonamide of the formula (4) with a halide of the formula (5) in the presence of a base.

The reaction of the sulfonyl chloride of the formula (2) with the aniline of the formula (3) is carried out according to the process disclosed in Japanese Unexamined Patent Publication Hei 4-145,060. Stated more specifically, as to the proportions of the sulfonyl chloride of the formula (2) and the aniline of the formula (3) used in the reaction, 0.5 to 2 moles, preferably 0.7 to 1.3 moles, of the latter is used per mole of the former. The reaction is conducted usually in an organic solvent. Useful organic solvents include a wide range of conventional organic solvents, e.g. aromatic hydrocarbons such as benzene, toluene and xylene, and halogenated hydrocarbons such as dichloromethane, chloroform and carbon tetrachloride, and mixtures thereof. This reaction is advantageously conducted in the presence of a basic compound such as β-picoline, γ-picoline or the like. The amount of the basic compound used is 0.01 to 1 mole, preferably 0.1 to 0.5 mole, per mole of the sulfonyl chloride of the formula (2). The reaction suitably proceeds at a temperature of 130 to 200°C, preferably 140 to 180°C, and is generally completed in about 3 to about 15 hours.

The reaction between the sulfonamide of the formula (4) and the halide of the formula (5) can be carried out usually in the presence of a base in a suitable solvent. Examples of useful solvents are ethers such as diethyl ether and tetrahydrofuran, nitriles such as acetonitrile and propionitrile, ketones such as acetone and methyl ethyl ketone, alcohols such as methanol and ethanol, and amides such as dimethylformamide and dimethylacetoamide, and mixtures thereof. Examples of useful bases are anhydrous carbonates of alkali metals such as sodium carbonate and potassium carbonate, hydroxides of alkali metals such as sodium hydroxide and potassium hydroxide, hydrides of alkali metals such as sodium hydride and potassium hydride, etc. These bases can be used either alone or in combination. The amount of such base used is about 1 to about 5 moles, preferably about 1 to about 2 moles, per mole of the sulfonamide of the formula (4). As to the proportions of the sulfonamide of the formula (4) and the halide of the formula (5) used in the reaction, about 0.5 to about 2 moles, preferably about 1 to about 1.2 moles, of the latter is used per mole of the former. The reaction properly proceeds at a temperature of 0°C to the boiling point of the solvent used, and is generally completed in about 1 to about 8 hours. The halide of the formula (5) to be used as the starting material in the reaction includes commercially available compounds.

The compound of the formula (1) according to the invention which is prepared by the foregoing process can be easily isolated and purified from the reaction mixture by conventional separation methods such as solvent extraction, recrystallization, column chromatography and the like.

According to the producing process shown in Reaction Scheme-1, the compound of the present invention can be prepared in a high yield and with high purity.

The compound of the present invention can effectively exterminate a wide range of noxious insects which pose an agricultural problem. Noxious insects against which the compound of the invention shows a particularly high insecticidal activity include the order Lepidoptera, e.g. Spodoptera litura, Plutella maculipennis, etc., the order Hemiptera, e.g. Myzus persicae, Laodelphax striatellus, etc., mites, e.g. Tetranychus urticae, Panonychus citri, etc., nematodes, e.g. Meloidogyne incognita Kofoid et White, etc.

For use as an insecticidal, miticidal and/or nematocidal agent, the compound of the invention is made available in the form of emulsions, hydrated compositions, aqueous solutions, particles, fine particles, granules, powders, coating compositions, spray preparations, aerosols, microcapsules, fumigants, fuming compositions, etc. When preparing these compositions, various surfactants are usable for emulsifying, dispersing, suspending or foaming purposes. Examples of useful surfactants include nonionic surfactants such as polyoxyethylene alkyl ethers, polyoxyethylene alkyl esters and polyoxyethylene sorbitan alkyl esters, and anionic surfactants such as alkylbenzenesulfonates, alkylsulfosuccinates, alkylsulfates, polyoxyethylene alkylsulfates, aryl sulfonates and lignin sulfites. Usable as solvents, diluents and carriers are various organic solvents, aerosol propellants, natural minerals, synthetic compounds, etc. Examples of preferred organic solvents are benzene, toluene, xylene, ethylbenzene, chlorobenzene, alkylnaphthalene, chloroethylene, cyclohexanone, methyl ethyl ketone, methyl isobutyl ketone, alcohol, cellosolve, dimethylformamide, dimethylsulfoxide, acetonitrile, mineral oil distillate, and water. Examples of useful aerosol propellants are propane, butane, hydrocarbon halides, nitrogen, carbon dioxide, etc. These compositions may be colored with an organic or inorganic dye.

The composition is prepared in a manner to incorporate the compound of the invention in an amount of about 0.1 to about 95% by weight, preferably about 0.5 to about 90% by weight.

The thus-obtained composition is used as such or as diluted with a carrier or water. In conformity with the contemplated purpose, the composition can be diluted to the range of about 0.0001 to 100% by weight as desired, preferably is diluted so as to contain 0.001 to 10% by weight of the active component.

### Best Mode for Carrying Out the Invention

Given below are Reference Examples illustrating the preparation of the starting compound as a raw material, Examples illustrating the preparation of the compound according to the invention and Test Examples to describe the invention in more detail.

### Reference Example 1

### Preparation of 3,5-dichloro-N-(4-trifluoromethylphenyl)benzenesulfonamide

A solution of 2.46 g (0.01 mole) of 3,5-dichlorophenyl benzenesulfonyl chloride in 5 ml of toluene was added dropwise to a solution of 1.61 g (0.01 mole) of p-aminobenzotrifluoride and 0.8 g (0.011 mole) of pyridine in 45 ml of toluene with stirring at up to 10°C. After addition, the reaction mixture was stirred at room temperature for 16 hours. Then, 100 ml of ethyl acetate was added after which the mixture was successively washed with a 5% aqueous solution of hydrochloric acid, a 5% aqueous solution of sodium hydrogencarbonate and an aqueous solution of sodium chloride and dried over anhydrous magnesium sulfate. The solvent was distilled off. The residual solids were washed with n-hexane, subjected to suction filtration and dried, giving the above-specified product (3.2 g, 85.8%) as white crystals.

### Reference Example 2

### Preparation of 2,4,5-trichloro-N-(4-trifluoromethylphenyl)benzenesulfonamide

A solution of 2.82 g (0.01 mole) of 2,4,5-trichlorophenyl benzenesulfonyl chloride in 5 ml of toluene was added dropwise to a solution of 1.61 g (0.01 mole) of p-aminobenzotrifluoride and 0.8 g (0.011 mole) of pyridine in 45 ml of toluene with stirring at up to 10°C. After addition, the reaction mixture was stirred at room temperature for 16 hours. Then, 100 ml of ethyl acetate was added after which the mixture was washed with a 5% aqueous solution of hydrochloric acid, a 5% aqueous solution of sodium hydrogencarbonate and an aqueous solution of sodium chloride and dried over anhydrous magnesium sulfate. The solvent was distilled off. The residual solids were washed with n-hexane, subjected to suction filtration and dried, giving the above-specified product in a yield of 73% as white crystals.

### Example 1

### Preparation of N-propargyl-3,5-dichloro-N-(4-trifluoromethylphenyl)benzenesulfonamide

A 2.22 g quantity (0.006 mole) of 3,5-dichloro-N-(4-trifluoromethylphenyl)benzenesulfonamide was added in small amounts to a suspension of 0.27 g (0.006 mole) of 60% sodium hydride in 15 ml of dimethylformamide at up to 10°C. After addition of the total amount, the reaction mixture was stirred at room temperature for 30 minutes after which a solution of 1.18 g (0.01 mole) of propargyl bromide in 5 ml of anhydrous benzene was added dropwise. After addition, the reaction mixture was stirred for a further 6 hours at room temperature. Cold water was poured into the reaction system, and the oil layer was extracted with benzene. The extract was washed with an aqueous solution of sodium chloride and dried over anhydrous magnesium sulfate. The solvent was distilled off and the residue was purified by silica gel column chromatography (n-hexane: ethyl acetate= 5:1), giving the above-specified product (2.1 g, 85.4%) as a yellow oil.
¹H-NMR(CDCl₃) δppm: 2.30 (1H, t), 4.48 (2H, d), 7.20-7.78 (7H, m)

Using proper starting materials, the compounds of Examples 2 to 45 as described below were prepared in the same manner as in Example 1.

### Example 2

Compound of the formula (1a) wherein R¹ and R² are hydrogen atoms, X is a hydrogen atom, and Y is a trifluoromethyl group (4-position):
Yellow oil
¹H-NMR (CDCl₃) δppm: 2.14 (1H, t), 4.36 (2H, d), 6.96-7.78 (9H, m)

### Example 3

Compound of the formula (1a) wherein R¹ and R² are hydrogen atoms, X is a hydrogen atom, and Y is a chlorine atom (4-position):
Melting point 81-82°C
¹H-NMR (CDCl₃) δppm: 2.18 (1H, t), 4.42 (2H, d), 7.20-7.80 (9H, m)

### Example 4

Compound of the formula (1a) wherein R¹ and R² are hydrogen atoms, X is a hydrogen atom, and Y is a chlorine atom (3-position and 5-position):
Melting point 99-101°C
¹H-NMR (CDCl₃) δppm: 2.24 (1H, t), 4.42 (2H, d), 6.82-7.90 (8H, m)

### Example 5

Compound of the formula (1a) wherein R¹ and R² are hydrogen atoms, X is a hydrogen atom, and Y is a fluorine atom (4-position):
Yellow oil
¹H-NMR (CDCl₃) δppm: 2.20 (1H, t), 4.42 (2H, d), 6.78-7.86 (9H, m)

### Example 6

Compound of the formula (1a) wherein R¹ and R² are hydrogen atoms, X is a hydrogen atom, and Y is a nitro group (4-position):
Yellow oil
¹H-NMR (CDCl₃) δppm: 2.24 (1H, t), 4.52 (2H, d), 7.24-8.32 (9H, m)

### Example 7

Compound of the formula (1a) wherein R¹ is a hydrogen atom, R² is a chlorine atom (2-position), X is a hydrogen atom, and Y is a nitro group (4-position):
Yellow oil
¹H-NMR (CDCl₃) δppm: 2.26 (1H, t), 4.48 (2H, d), 7.20-8.38 (8H, m)

### Example 8

Compound of the formula (1a) wherein R¹ and R² are hydrogen atoms, X is a chlorine atom (4-position), and Y is a trifluoromethyl group (4-position):
Melting point 67-68°C
¹H-NMR (CDCl₃) δppm: 2.24 (1H, t), 4.48 (2H, d), 7.22-7.78 (8H, m)

### Example 9

Compound of the formula (1a) wherein R¹ and R² are hydrogen atoms, X is a chlorine atom (3-position), and Y is a trifluoromethyl group (4-position):
Yellow oil
¹H-NMR (CDCl₃) δppm: 2.30 (1H, t), 4.48 (2H, d), 7.02-7.82 (8H, m)

### Example 10

Compound of the formula (1a) wherein R¹ and R² are hydrogen atoms, X is a methoxy group (4-position), and Y is a trifluoromethyl group (4-position):
Yellow Oil
¹H-NMR (CDCl₃) δppm: 2.22 (1H, t), 3.80 (3H, s), 4.48 (2H, d), 7.02-7.82 (8H, m)

### Example 11

Compound of the formula (1a) wherein R¹ and R² are hydrogen atoms, X is a cyano group (4-position), and Y is a trifluoromethyl group (4-position):
Melting point 108-110°C
¹H-NMR (CDCl₃) δppm: 2.26 (1H, t), 4.48 (2H, d), 7.24-7.76 (8H, m)

### Example 12

Compound of the formula (1a) wherein R¹ and R² are hydrogen atoms, X is a tert-butyl group (4-position), and Y is a trifluoromethyl group (4-position):
Yellow oil
¹H-NMR (CDCl₃) δppm: 1.30 (9H, s), 2.26 (1H, d), 4.48 (2H, d), 7.24-7.76 (8H, m)

### Example 13

Compound of the formula (1a) wherein R¹ and R² are hydrogen atoms, X is a nitro group (4-position), and Y is a trifluoromethyl group (4-position):
Yellow oil
¹H-NMR (CDCl₃) δppm: 2.26 (1H, t), 4.52 (2H, d), 7.26-8.48 (8H, m)

### Example 14

Compound of the formula (1a) wherein R¹ and R² are hydrogen atoms, X is a chlorine atom (3-position and 4-position), and Y is a trifluoromethyl group (4-position):
Melting point 97-99°C
¹H-NMR (CDCl₃) δppm: 2.28 (1H, t), 4.48 (2H, d), 7.20-7.90 (7H, m)

### Example 15

Compound of the formula (1a) wherein R¹ and R² are hydrogen atoms, X is a chlorine atom (2-position, 4-position and 5-position) and Y is a trifluoromethyl group (4-position):
Yellow oil
¹H-NMR (CDCl₃) δppm: 2.30 (1H, t), 4.58 (2H, d), 7.38-7.78 (5H, m), 8.0 (1H, s)

### Example 16

Compound of the formula (1a) wherein R¹ and R² are hydrogen atoms, X is a chlorine atom (3-position and 5-position), and Y is a nitro group (4-position):
Melting point 96-97°C
¹H-NMR (CDCl₃) δppm: 2.32 (1H, t), 4.50 (2H, d), 7.38-8.32 (7H, m)

### Example 17

Compound of the formula (1a) wherein R¹ is a hydrogen atom, R² is a fluorine atom (4-position), X is a chlorine atom (3-position and 5-position), and Y is a nitro group (3-position):
Melting point 94-95°C
¹H-NMR (CDCl₃) δppm: 2.26 (1H, t), 4.50 (2H, d), 7.12-7.96 (6H, m)

### Example 18

Compound of the formula (1a) wherein R¹ is a hydrogen atom, R² is a chlorine atom (4-position), X is a chlorine atom (3-position and 5-position), and Y is a trifluoromethyl group (3-position):
Yellow oil
¹H-NMR (CDCl₃) δppm: 2.33 (1H, t), 4.48 (2H, d), 7.26-7.80 (6H, m)

### Example 19

Compound of the formula (1a) wherein R¹ and R² are hydrogen atoms, X is a chlorine atom (3-position and 5-position), and Y is a trifluoromethyl group (3-position):
Yellow oil
¹H-NMR (CDCl₃) δppm: 2.34 (1H, t), 4.48 (2H, d), 7.20-7.80 (7H, m)

### Example 20

Compound of the formula (1a) wherein R¹ and R² are hydrogen atoms, X is a chlorine atom (3-position and 5-position), and Y is a fluorine atom (3-position and 4-position):
Melting point 97-98°C
¹H-NMR (CDCl₃) δppm: 2.32 (1H, t), 4.42 (2H, d), 6.92-7.62 (6H, m)

### Example 21

Compound of the formula (1a) wherein R¹ and R² are hydrogen atoms, X is a chlorine atom (3-position and 5-position), and Y is a fluorine atom (2-position and 4-position):
Melting point 81-82°C
¹H-NMR (CDCl₃) δppm: 2.28 (1H, t), 4.40 (2H, d), 6.62-7.22 (6H, m)

### Example 22

Compound of the formula (1a) wherein R¹ and R² are hydrogen atoms, X is a chlorine group (3-position and 5-position), and Y is a trifluoromethoxy group (4-position):
Melting point 87-89°C
¹H-NMR (CDCl₃) δppm: 2.32 (1H, t), 4.48 (2H, d), 7.06-7.70 (7H, m)

### Example 23

Compound of the formula (1a) wherein R¹ and R² are hydrogen atoms, X is a chlorine atom (2-position, 4-position and 5-position), and Y is a trifluoromethyl group (3-position):
Yellow oil
¹H-NMR (CDCl₃) δppm: 2.32 (1H, t), 4.62 (2H, d), 7.20-8.22 (6H, m)

### Example 24

Compound of the formula (1a) wherein R¹ is a hydrogen atom, R² is a chlorine atom (4-position), X is a chlorine atom (2-position, 4-position and 5-position), and Y is a trifluoromethyl group (3-position):
Yellow oil
¹H-NMR (CDCl₃) δppm: 2.30 (1H, t), 4.52 (2H, d), 7.38-8.10 (5H, m)

### Example 25

Compound of the formula (1a) wherein R¹ and R² are hydrogen atoms, X is a chlorine atom (2-position, 4-position and 5-position), and Y is a fluorine atom (2-position and 4-position):
Melting point 72-73°C
¹H-NMR (CDCl₃) δppm: 2.34 (1H, t), 4.62 (2H, d), 6.62-8.04 (5H, m)

### Example 26

Compound of the formula (1a) wherein R¹ and R² are hydrogen atoms, X is a chlorine atom (2-position, 4-position and 5-position) and Y is a fluorine atom (3-position and 4-position):
Melting point 83-84°C
¹H-NMR (CDCl₃) δppm: 2.30 (1H, t), 4.52 (2H, d), 6.80-8.06 (5H, m)

### Example 27

Compound of the formula (1a) wherein R¹ and R² are hydrogen atoms, X is a chlorine atom (2-position, 4-position and 5-position), and Y is a trifluoromethyl group (3-position and 5-position):
Melting point 99-100°C
¹H-NMR (CDCl₃) δppm: 2.40 (1H, t), 4.60 (2H, d), 6.40-8.26 (5H, m)

### Example 28

Compound of the formula (1a) wherein R¹ and R² are hydrogen atoms, X is a chlorine atom (3-position and 4-position), and Y is a trifluoromethyl group (3-position):
Yellow oil
¹H-NMR (CDCl₃) δppm: 2.26 (1H, t), 4.46 (2H, d), 7.26-7.90 (7H, m)

### Example 29

Compound of the formula (1a) wherein R¹ is a hydrogen atom, R² is a chlorine atom (4-position), X is a chlorine atom (3-position and 4-position), and Y is a trifluoromethyl group (3-position):
Melting point 112-114°C
¹H-NMR (CDCl₃) δppm: 2.30 (1H, t), 4.44 (2H, d), 7.28-7.90 (6H, m)

### Example 30

Compound of the formula (1a) wherein R¹ and R² are hydrogen atoms, X is a chlorine atom (3-position and 4-position), and Y is a fluorine atom (2-position and 4-position):
Melting point 90-91°C
¹H-NMR (CDCl₃) δppm: 2.28 (1H, t), 4.42 (2H, d), 6.68-7.96 (6H, m)

### Example 31

Compound of the formula (1a) wherein R¹ and R² are hydrogen atoms, X is a chlorine atom (3-position and 4-position), and Y is a trifluoromethoxy group (4-position):
Melting point 101-102°C
¹H-NMR (CDCl₃) δppm: 2.24 (1H, t), 4.40 (2H, d), 7.06-7.78 (7H, m)

### Example 32

Compound of the formula (1a) wherein R¹ is a hydrogen atom, R² is a fluorine atom (4-position), X is a chlorine atom (3-position, 4-position and 5-position), and Y is a nitro group (3-position):
Melting point 118-119°C
¹H-NMR (CDCl₃) δppm: 2.32 (1H, t), 4.56 (2H, d), 7.06-8.14 (5H, m)

### Example 33

Compound of the formula (1a) wherein R¹ and R² are hydrogen atoms, X is a chlorine atom (2-position and 5-position), and Y is a trifluoromethyl group (4-position):
Yellow oil
¹H-NMR (CDCl₃) δppm: 2.30 (1H, t), 4.62 (2H, d), 7.22-8.02 (7H, m)

### Example 34

Compound of the formula (1a) wherein R¹ and R² are hydrogen atoms, X is a trifluoromethyl group (3-position), and Y is a trifluoromethyl group (4-position):
Yellow oil
¹H-NMR (CDCl₃) δppm: 2.23 (1H, t), 4.48 (2H, d), 7.20-8.10 (8H, m)

### Example 35

Compound of the formula (1a) wherein R¹ and R² are hydrogen atoms, X is a trifluoromethyl group (3-position and 5-position), and Y is a trifluoromethyl group (4-position):
Melting point 128-130°C
¹H-NMR (CDCl₃) δppm: 2.42 (1H, t), 4.56 (2H, d), 7.10-8.40 (8H, m)

### Example 36

Compound of the formula (1a) wherein R¹ and R² are hydrogen atoms, X is a fluorine atom (4-position) and Y is a trifluoromethyl group (4-position):
Melting point 57-58°C
¹H-NMR (CDCl₃) δppm: 2.22 (1H, t), 4.48 (2H, d), 6.94-7.90 (8H, m)

### Example 37

Compound of the formula (1a) wherein R¹ and R² are hydrogen atoms, X is a fluorine atom (4-position), and Y is a fluorine atom (2-position and 6-position):
Yellow oil
¹H-NMR (CDCl₃) δppm: 2.18 (1H, t), 4.36 (2H, d), 6.70-8.10 (6H, m)

### Example 38

Compound of the formula (1a) wherein R¹ and R² are hydrogen atoms, X is a cyano group (4-position), and Y is a trifluoromethyl group (3-position):
Melting point 103-104°C
¹H-NMR (CDCl₃) δppm: 2.30 (1H, t), 4.50 (2H, d), 7.30-7.88 (4H, m)

### Example 39

Compound of the formula (1a) wherein R¹ is a hydrogen atom, R² is a chlorine atom (4-position), X is a cyano group (4-position), and Y is a trifluoromethyl group (3-position): Melting point 135-137°C
¹H-NMR (CDCl₃) δppm: 2.32 (1H, t), 4.48 (2H, d), 7.38-7.68 (3H, m), 7.78 (4H, s)

### Example 40

Compound of the formula (1a) wherein R¹ and R² are hydrogen atoms, X is a cyano group (4-position), and Y is a fluorine atom (3-position and 4-position):
Melting point 116-118°C
¹H-NMR (CDCl₃) δppm: 2.26 (1H, t), 4.46 (2H, d), 6.98-7.80 (6H, m)

### Example 41

Compound of the formula (1a) wherein R¹ and R² are hydrogen atoms, X is a chlorine atom (3-position and 5-position), and Y is 4-trifluoromethyl-2-pyridyloxy group (4-position):
Melting point 108-111°C
¹H-NMR (CDCl₃) δppm: 2.20 (1H, t), 4.42 (2H, d), 6.90-7.95 (9H, m), 8.35 (1H, bs)

### Example 42

Compound of the formula (1a) wherein R¹ and R² are hydrogen atoms, X is a chlorine atom (3-position and 4-position), and Y is (4-trifluoromethyl)-2-pyridyloxy group (4-position):
Melting point 140-141°C
¹H-NMR (CDCl₃) δppm: 2.32 (1H, t), 4.42 (2H, d), 7.00-8.10 (9H, m), 8.40 (1H, bs)

### Example 43

Compound of the formula (1a) wherein R¹ is a trifluoromethyl group (3-position), R² is a hydrogen atom, X is a chlorine atom (4-position), and Y is a trifluoromethyl group (3-position and 5-position):
Yellow oil
¹H-NMR (CDCl₃) δppm: 2.30 (1H, t), 4.48 (2H, d), 7.02-7.82 (7H, m)

### Example 44

Compound of the formula (1a) wherein R¹ and R² are hydrogen atoms, X is a trifluoromethyl group (3-position and 5-position), Y is a trifluoromethyl group (3-position and 5-position):
Yellow oil
¹H-NMR (CDCl₃) δppm: 2.28 (1H, t), 4.46 (2H, d), 7.02-7.92 (7H, m)

### Example 45

Compound of the formula (1a) wherein R¹ and R² are hydrogen atoms, X is a chlorine atom (3-position and 5-position), and Y is a trifluoromethyl group (3-position and 5-position):
Yellow oil
¹H-NMR (CDCl₃) δppm: 2.30 (1H, t), 4.43 (2H, d), 6.98-7.88 (7H, m)

### Example 46

### Preparation of N-cyanomethyl-2,4,5-trichloro-N-(4-trifluoromethylphenyl)benzenesulfonamide

A 2.35 g quantity (0.006 mole) of 2,4,5-trichloro-N-(4-trifluoromethylphenyl)benzenesulfonamide was added in small amounts to a suspension of 0.27 g (0.006 mole) of 60% sodium hydride in 15 ml of dimethylformamide at up to 10°C. After addition of the total amount, the reaction mixture was stirred at room temperature for 30 minutes after which a solution of 1.20 g (0.01 mole) of bromoacetonitrile in 5 ml of anhydrous benzene was added dropwise. After addition, the reaction mixture was stirred for a further 5 hours at room temperature. Cold water was poured into the reaction system, and the oil layer was extracted with benzene. The extract was washed with an aqueous solution of sodium chloride and dried over anhydrous magnesium sulfate. The solvent was distilled off and the residue was purified by silica gel column chromatography (benzene: ethyl acetate= 20:1), giving the above-specified product (3.02 g, 70%) as a pale yellow oil.
¹H-NMR(CDCl₃) δppm: 4.90 (2H), 7.62-8.02 (6H)

Using proper starting materials, the compounds of Examples 47 to 65 as described below were prepared in the same manner as in Example 46.

### Example 47

Compound of the formula (1b) wherein R^{3'} is an ethoxy group, X' is a chlorine atom (3-position and 5-position) and Y' is a fluorine atom (3-position and 4-position):
Yellow oil
¹H-NMR (CDCl₃) δppm: 1.22 (3H), 3.52 (2H), 4.96 (2H), 6.92-7.70 (6H)

### Example 48

Compound of the formula (1b) wherein R^{3'} is an ethoxy group, X' is a chlorine atom (3-position and 4-position) and Y' is a trifluoromethyl group (3-position and 5-position):
Yellow oil
¹H-NMR (CDCl₃) δppm: 1.22 (3H), 3.58 (2H), 5.04 (2H), 7.38-8.10 (6H)

### Example 49

Compound of the formula (1b) wherein R^{3'} is an ethoxy group, X' is a chlorine atom (3-position and 4-position) and Y' is a chlorine atom (4-position) and a trifluoromethyl group (5-position):
Yellow oil
¹H-NMR (CDCl₃) δppm: 1.22 (3H), 3.58 (2H), 5.04 (2H), 7.28-7.90 (6H)

### Example 50

Compound of the formula (1b) wherein R^{3'} is an ethoxy group, X' is a chlorine atom (2-position, 4-position and 5-position) and Y' is a fluorine atom (3-position and 4-position):
Yellow oil
¹H-NMR (CDCl₃) δppm: 1.22 (3H), 3.62 (2H), 5.14 (2H), 6.82-7.96 (5H)

### Example 51

Compound of the formula (1b) wherein R^{3'} is an ethoxy group, X' is a cyano group (4-position) and Y' is a trifluoromethyl group (3-position and 5-position):
Yellow oil
¹H-NMR (CDCl₃) δppm: 1.20 (3H), 3.52 (2H), 5.02 (2H), 7.20-7.98 (7H)

### Example 52

Compound of the formula (1b) wherein R^{3'} is an ethoxy group, X' is a chlorine atom (2-position, 4-position and 5-position) and Y' is a trifluoromethyl group (3-position and 5-position):
Yellow oil
¹H-NMR (CDCl₃) δppm: 1.24 (3H), 3.68 (2H), 5.20 (2H), 7.60-8.08 (5H)

### Example 53

Compound of the formula (1b) wherein R^{3'} is a methoxy group, X' is a trifluoromethyl group (3-position and 5-position) and Y' is a trifluoromethyl group (4-position):
Yellow oil
¹H-NMR (CDCl₃) δppm: 3.42 (3H), 4.96 (2H), 7.02-8.46 (7H)

### Example 54

Compound of the formula (1b) wherein R^{3'} is an ethoxy group, X' is a trifluoromethyl group (3-position and 5-position) and Y' is a trifluoromethyl group (4-position):
Yellow oil
¹H-NMR (CDCl₃) δppm: 1.20 (3H), 3.62 (2H), 5.20 (2H), 7.02-8.42 (7H)

### Example 55

Compound of the formula (1b) wherein R^{3'} is a cyano group, X' is a chlorine atom (3-position and 5-position), Y' is a chlorine atom (4-position) and a trifluoromethyl group (5-position):
Melting point 113-115°C
¹H-NMR (CDCl₃) δppm: 4.76 (2H), 7.32-7.90 (6H)

### Example 56

Compound of the formula (1b) wherein R^{3'} is a cyano group, X' is a chlorine atom (2-position, 4-position and 5-position), Y' is a trifluoromethyl group (3-position and 5-position):
Melting point 117-119°C
¹H-NMR (CDCl₃) δppm: 4.90 (2H), 7.42-8.26 (5H)

### Example 57

Compound of the formula (1b) wherein R^{3'} is a cyano group, X' is a trifluoromethyl group (3-position and 5-position), and Y' is a trifluoromethyl group (4-position):
Melting point 124-126°C
¹H-NMR (CDCl₃) δppm: 4.90 (2H), 7.02-8.10 (7H)

### Example 58

Compound of the formula (1b) wherein R^{3'} is a cyano group, X' is a trifluoromethyl group (3-position and 5-position), and Y' is a cyano group (4-position):
Yellow paste
¹H-NMR (CDCl₃) δppm: 4.92 (2H), 6.98-8.12 (7H)

### Example 59

Compound of the formula (1b) wherein R^{3'} is a cyano group, X' is a trifluoromethyl group (3-position and 5-position) and Y' is a nitro group (4-position):
Yellow paste
¹H-NMR (CDCl₃) δppm: 4.92 (2H), 7.04-8.26 (6H)

### Example 60

Compound of the formula (1b) wherein R^{3'} is a cyano group, X' is a trifluoromethyl group (3-position and 5-position), and Y' is a trifluoromethyl group (3-position and 5-position):
Yellow oil
¹H-NMR (CDCl₃) δppm: 4.90 (3H), 7.20-8.42 (6H)

### Example 61

Compound of the formula (1b) wherein R^{3'} is a cyano group, X' is a trifluoromethyl group (4-position) and Y' is a chlorine atom (2-position) and a nitro group (4-position):
Yellow paste
¹H-NMR (CDCl₃) δppm: 4.90 (2H), 7.22-8.38 (7H)

### Example 62

Compound of the formula (1b) wherein R^{3'} is a cyano group, X' is a trifluoromethyl group (4-position) and Y' is a trifluoromethyl group (4-position):
Yellow oil
¹H-NMR (CDCl₃) δppm: 4.90 (2H), 7.28-8.43 (8H)

### Example 63

Compound of the formula (1b) wherein R^{3'} is an ethoxycarbonyl group, X' is a chlorine atom (2-position, 4-position and 5-position) and Y' is a trifluoromethyl group (3-position and 5-position):
Yellow paste
¹H-NMR (CDCl₃) δppm: 1.20 (3H), 4.10 (2H), 4.50 (2H), 7.60-8.06 (5H)

### Example 64

Compound of the formula (1b) wherein R^{3'} is an ethoxycarbonyl group, X' is a trifluoromethyl group (3-position and 5-position) and Y' is a trifluoromethyl group (4-position):
Yellow oil
¹H-NMR (CDCl₃) δppm: 1.22 (3H), 4.10 (2H), 4.52 (2H), 7.04-8.40 (7H)

### Example 65

Compound of the formula (1b) wherein R^{3'} is a methoxycarbonyl group, X' is a trifluoromethyl group (3-position and 5-position) and Y' is a trifluoromethyl group (4-position):
Yellow oil
¹H-NMR (CDCl₃) δppm: 3.98 (3H), 4.52 (2H), 7.02-8.40 (7H)

### Test Example 1 (test on two-spotted spider mites)

Two parts by weight of the compound of the invention was dissolved in 98 parts by weight of acetone. The solution was diluted to a predetermined concentration with an aqueous solution of a 0.04% spreader (trademark "Shinrino", product of Nihon Nohyaku Co., Lt.). Adult two-spotted spider mites (Tetranychus urticae Koch) were placed on kidney-bean plants in pots and the above dilution was sprayed onto the plant until it dripped. The mortality was determined 4 days later. The results are shown in Table 1. The test compound No. in Table 1 correspond to Example No. (the same in Tables 2 and 3).

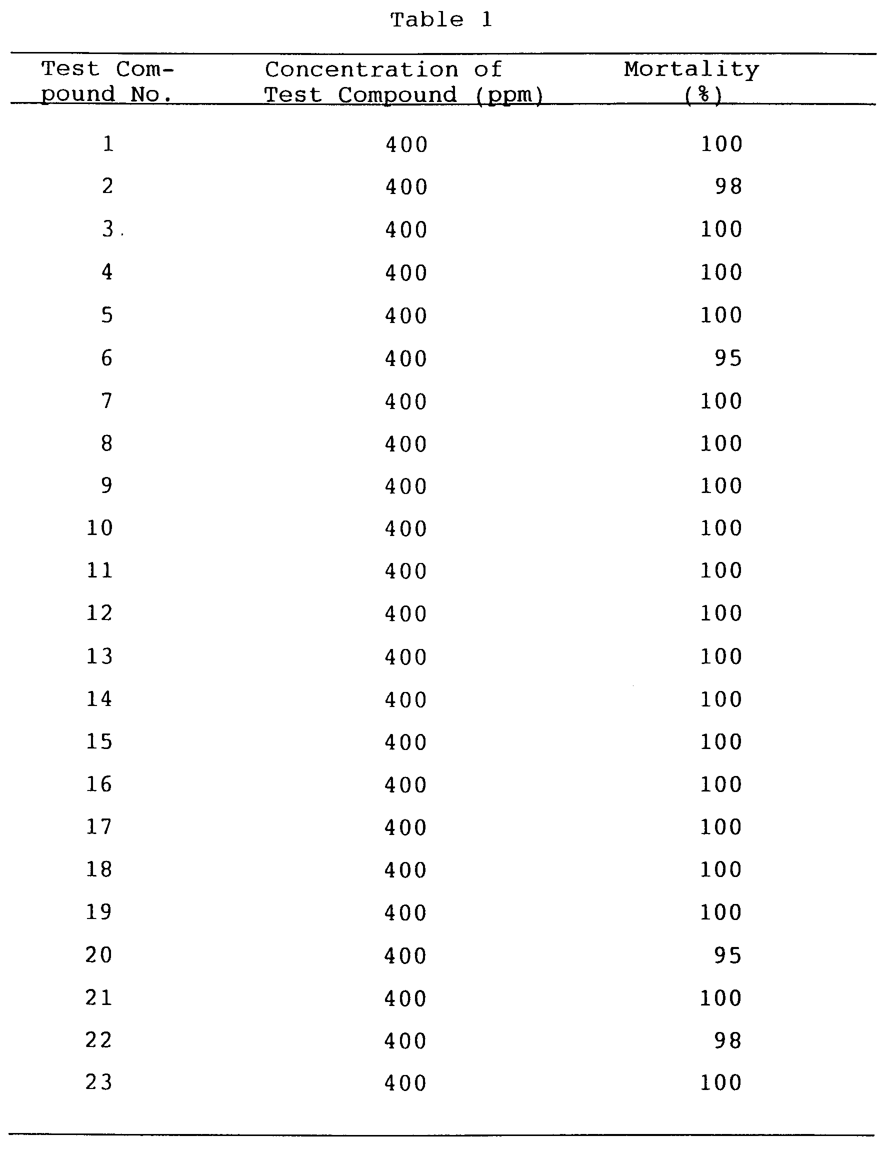

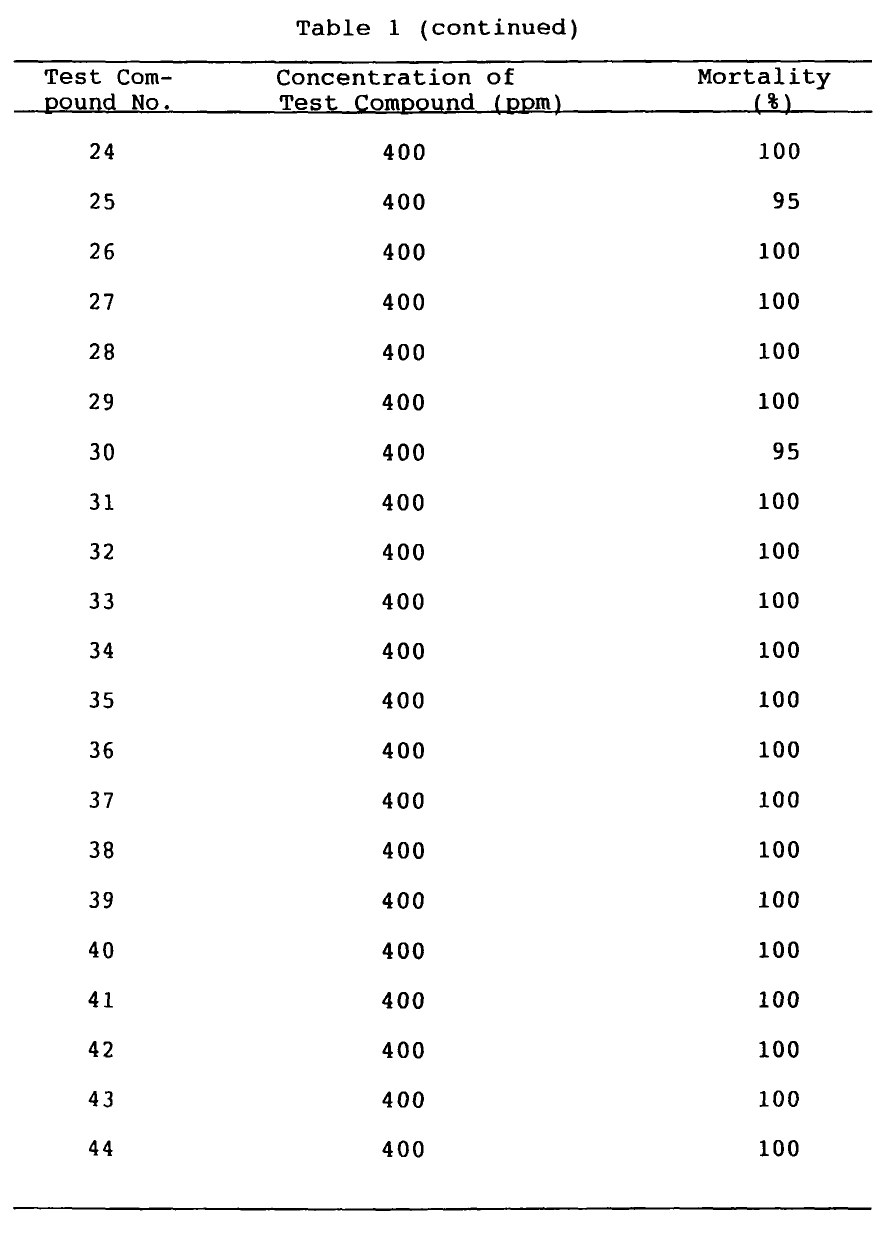

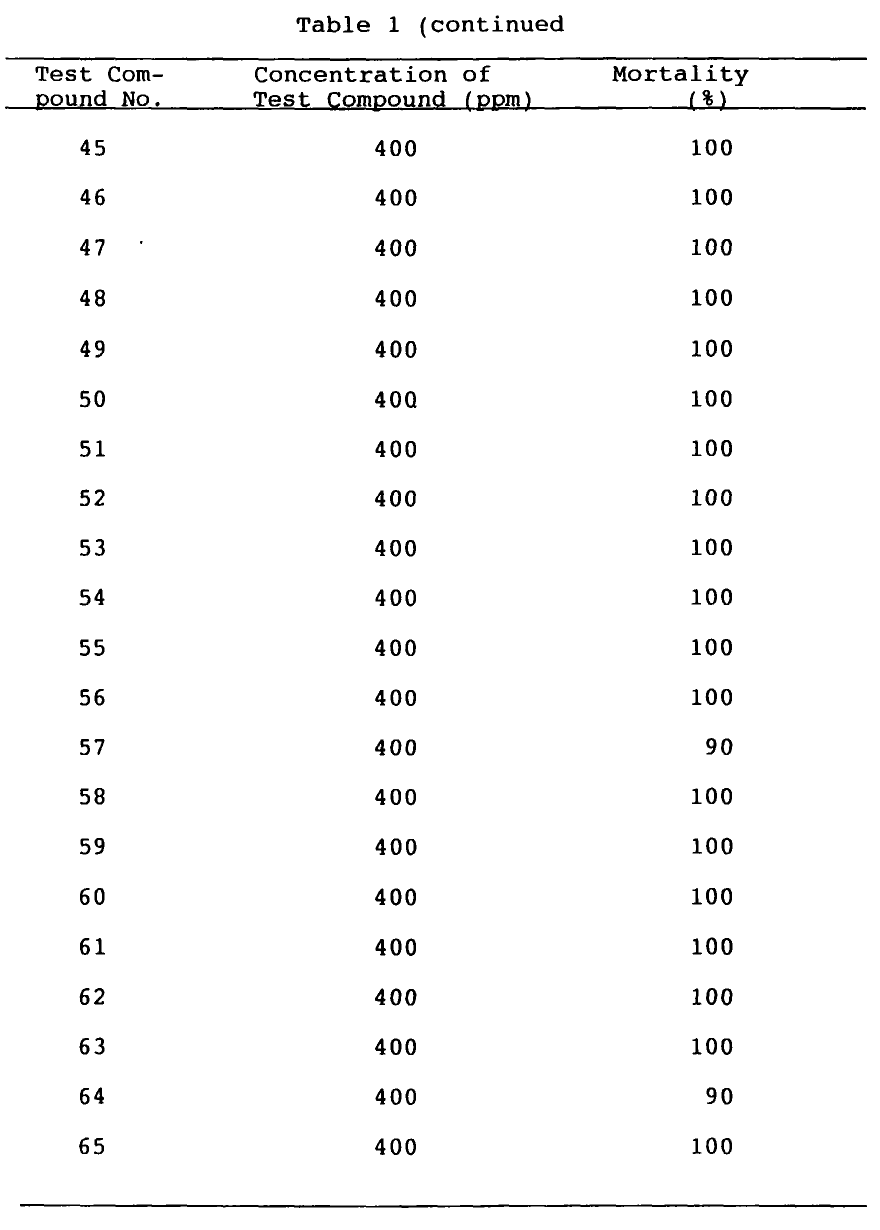

### Test Example 2 (test on diamond-back moth)

Two parts by weight of the compound of the invention was dissolved in 98 parts by weight of acetone. The solution was diluted to a predetermined concentration with an aqueous solution of a 0.04% spreader (trademark "Shinrino", product of Nihon Nohyaku Co., Lt.). The leaves of cabbages (7 X 7 cm) were dipped in the solution for 10 seconds. Fourth-instar larvae of diamond-back moth (Plutella xylostella Linné) were placed, together with the leaves thus treated, into a cup of plastics 13 cm in diameter. The cup was left to stand in a thermostatic chamber maintained at 25 ± 1°C. The insects were checked for mortality and for emergence control ratio (wing growth control ratio) 2 days and 7 days after treatment, respectively. The results are shown in Table 2. The emergence control ratio was evaluated according to the following grades: A: 100%, B: 90 (inclusive)-100 (exclusive)%, C: 50 (inclusive) to 90 (exclusive)%, D: less than 50%

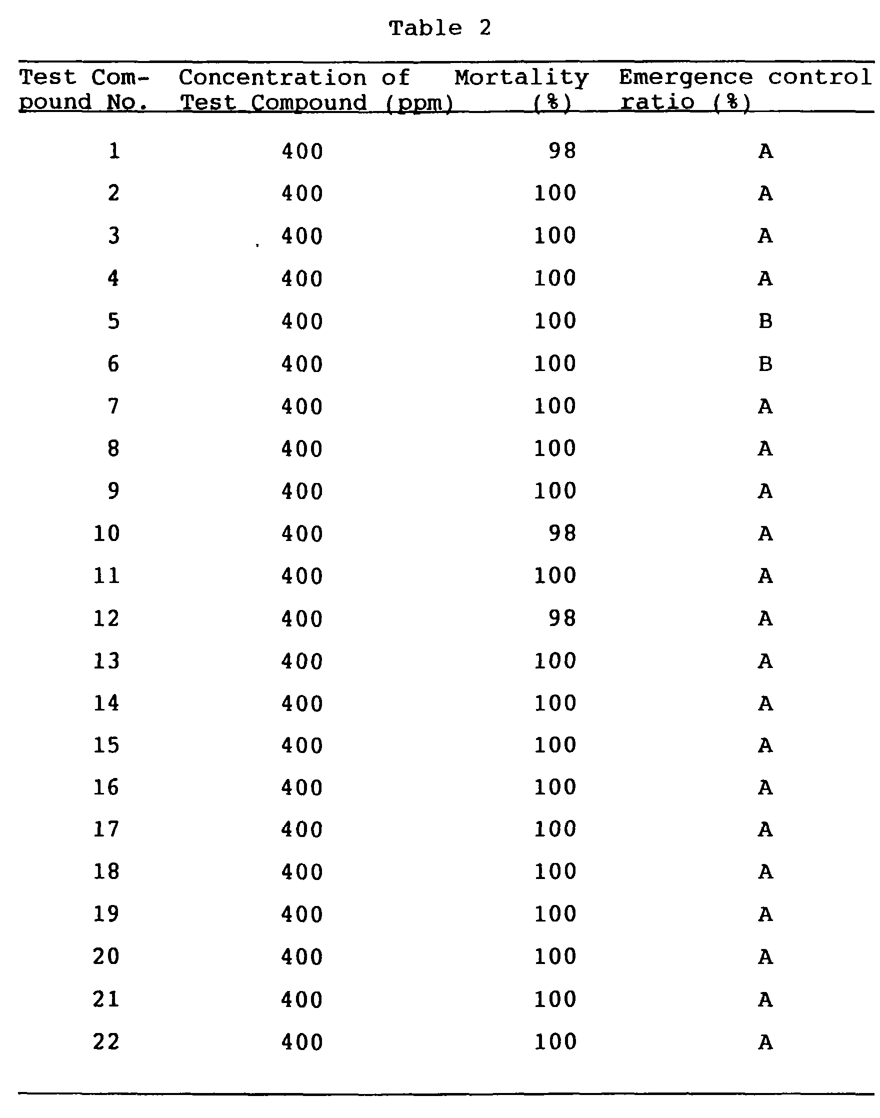

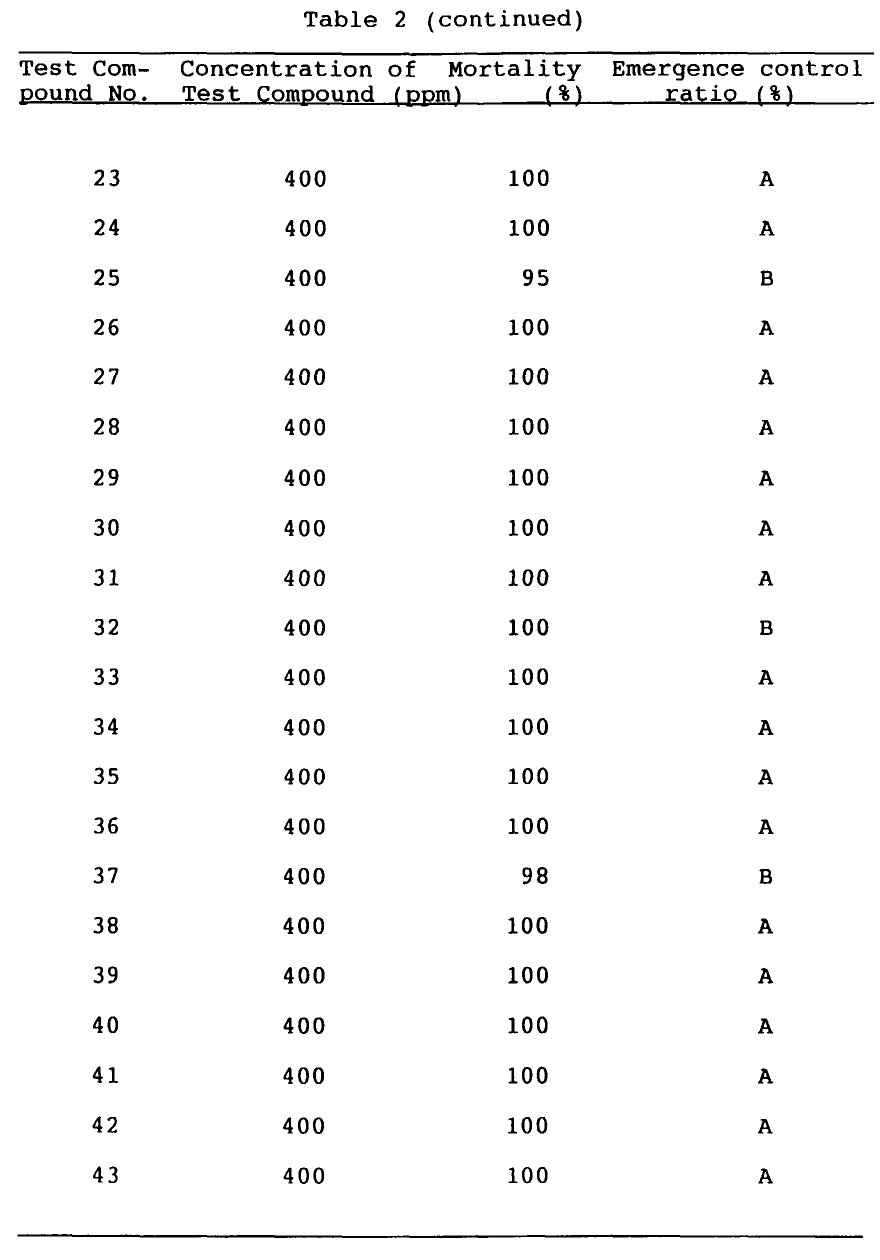

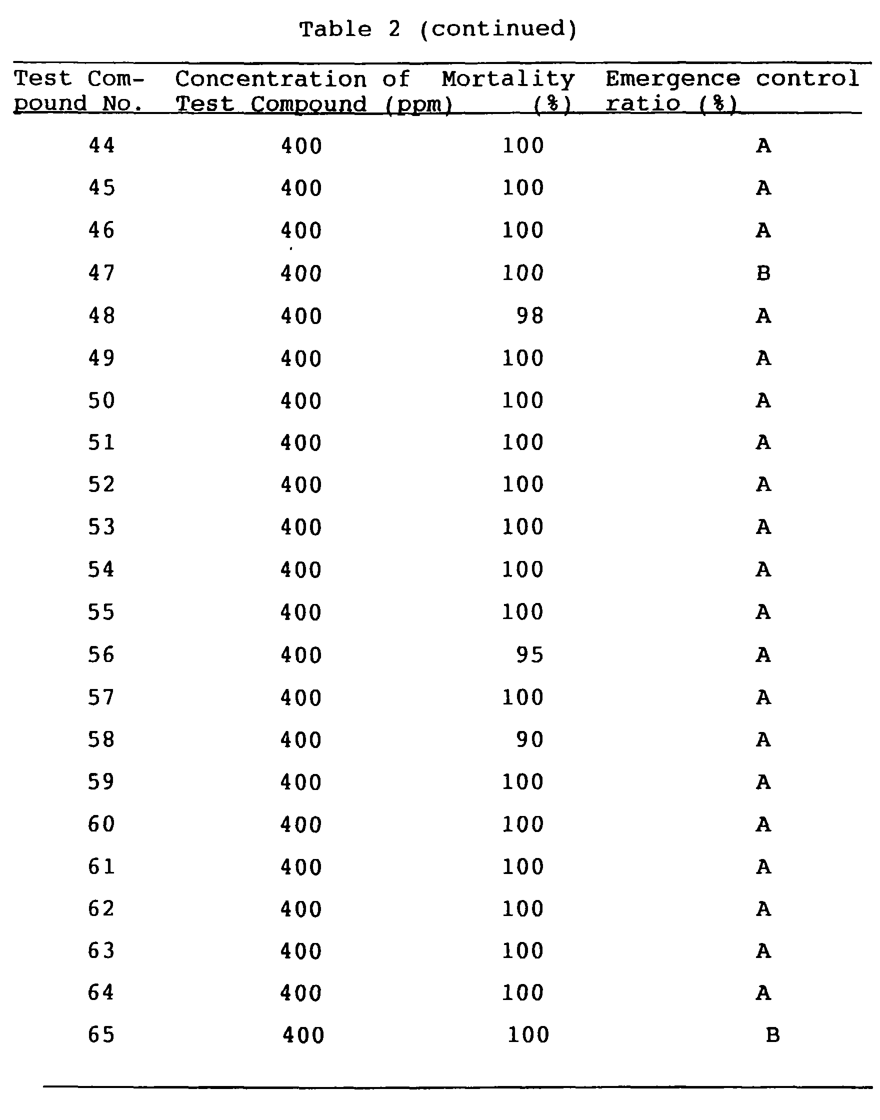

### Test Example 3 (test on southern root-knot nematode)

A 1 mg quantity of the compound of the invention was dissolved in 100 µl of acetone. The solution was added to 9900 µl of an aqueous solution containing 0.1% Tween 80 to give 10 ml of a solution.

Into a 10-ml sample bottle were placed 8 g of 100-20 mesh sand (river sand) dried at 120°C for 3 hours, 1 ml of a nematode suspension containing 500 2nd-instar larvae of southern root-knot nematode (Meloidogyne incognita Kofoid et White) (L2) and 1 ml of said solution (concentration of present compound 100 ppm) in this order. The mixture was left to stand in a thermostatic container (dark) maintained at 25°C for 24 hours. Thereafter tomato seedlings (1-leaf stage) with the roots cut off were inserted into the sand to a depth of several millimeters and left to stand at 25°C in an assay room. Fourteen days later, the roots were washed with water and the number of root knots was counted to determine the control ratio.

**Table 3**

| Test Compound No. | Concentration of Test Compound (ppm) | Control ratio (%) |
|---|---|---|
| 5 | 100 | 100 |
| 16 | 100 | 100 |
| 17 | 100 | 100 |
| 18 | 100 | 100 |
| 19 | 100 | 100 |
| 20 | 100 | 100 |
| 30 | 100 | 100 |
| 31 | 100 | 100 |
| 32 | 100 | 100 |
| 36 | 100 | 100 |
| 37 | 100 | 100 |
| 38 | 100 | 100 |
| 47 | 100 | 100 |
| 48 | 100 | 98 |
| 49 | 100 | 100 |
| 50 | 100 | 100 |
| 54 | 100 | 98 |

## Claims

1. A sulfonamide derivative represented by the formula 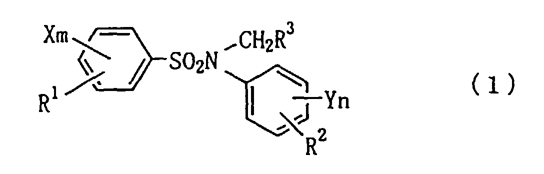 wherein R¹ and R² are the same or different and each represents a hydrogen atom, a lower haloalkyl group or a halogen atom, R³ is an ethynyl group, a cyano group, a lower alkoxy group, or a lower alkoxycarbonyl group, X and Y are the same or different and each represents a hydrogen atom, a halogen atom, a lower alkyl group, a lower alkoxy group, a lower haloalkyl group, a lower haloalkoxy group, a phenoxy group optionally having a substituent(s), a phenyl group optionally having a substituent(s), a pyridyloxy group optionally having a substituent(s), a cyano group, a nitro group, a lower alkylthio group or a lower alkyl sulfonyl group, and m and n are each an integer of 1 to 3.

2. An insecticidal, miticidal or nematocidal composition containing the sulfonamide derivative of the formula (1) defined in claim 1.
